# EUROPÄISCHE PATENTSCHRIFT

(11) **EP 1 372 758 B1**
(45) Veröffentlichungstag und Bekanntmachung des Hinweises auf die Patenterteilung: **09.07.2008**
(21) Anmeldenummer: 02759794.7
(22) Anmeldetag: 05.04.2002
(51) Int. Cl.: A61M 1/34, A61M 1/36, A61K 9/127

(54) **VERFAHREN ZUR ELIMINIERUNG VON POTENTIELL TOXISCHEN UND/ODER SCHÄDLICHEN STOFFEN**
METHOD FOR ELIMINATING POTENTIALLY TOXIC AND/OR HARMFUL SUBSTANCES
PROCEDE POUR ELIMINER DES SUBSTANCES POTENTIELLEMENT TOXIQUES ET/OU NOCIVES

(30) Priorität: 05.04.2001 DE 10117043
(43) Veröffentlichungstag der Anmeldung: 02.01.2004
(73) Patentinhaber: Eckes, Dr., Jürgen, 79312 Kollmarsreute (DE); Pütz, Dr., Gerhard, 79206 Breisach (DE)
(72) Erfinder: PÜTZ, Gerhard, 79115 Freiburg (DE); ECKES, Jürgen, 79115 Freiburg (DE)
(74) Vertreter: Oser, Andreas
(86) Internationale Anmeldenummer: PCT/EP2002/003800
(87) Internationale Veröffentlichungsnummer: WO 2002/081006

(56) Entgegenhaltungen:
- EP-A- 0 120 445
- EP-A- 0 361 894
- GB-A- 2 232 984
- US-A- 4 643 718
- US-A- 5 622 713
- DATABASE BIOSIS [Online] BIOSCIENCES INFORMATION SERVICE, PHILADELPHIA, PA, US; 15. Mai 1999 (1999-05-15) CHOICE E ET AL: "Separation of liposomes from plasma components using fast protein liquid chromatography." Database accession no. PREV199900273829 XP002206315 in der Anmeldung erwähnt & ANALYTICAL BIOCHEMISTRY, Bd. 270, Nr. 1, 15. Mai 1999 (1999-05-15), Seiten 1-8, ISSN: 0003-2697
- YUMI T. ET AL: 'Ability to remove immunoglobulins...' J OF NEUROLOGICAL SCIENCES Bd. 157, 1998, Seiten 90 - 95
- JUNJI K. ET AL: 'Reduction of lipoprotein (a)...' ATHEROSCLEROSIS Bd. 100, 1993, Seiten 65 - 74

## Beschreibung

Die vorliegende Erfindung betrifft eine Verwendung von potentiell toxischen und/oder schädlichen Wirkstoff enthaltenden Partikeln zur Herstellung eines Medikaments. Die Erfindung betrifft insbesondere eine solche Verwendung während einer oder im Anschluss an eine medizinische Therapie, bei der ein therapeutischer Stoff durch vollständige oder teilweise Eliminierung beseitigt, reduziert oder in der Dosis gesteuert wird.

Therapeutische Wirkstoffe können cytotoxisch oder auf andere Weise toxisch wirken. So werden häufig, z.B. im Rahmen maligner (Tumor-) Erkrankungen oder Infektionen, mit auch für gesunde Körpergewebe bzw. -organe hochtoxischen Wirkstoffen medizinische Therapien durchgeführt. Neben den Chemotherapeutika bzw. Cytostatika kann dies auch auf andere Antikrebsmitteln, Antibiotika, Antivirusmitteln, Antimalariamitteln, Antimykotika, Interferone, Cytokine usw. zutreffen. Die Applikation erfolgt häufig systemisch, insbesondere intravenös. Durch eine meist wenig selektive Bindung bzw. Aufnahme in Zellen oder einem anderen Zielort entfaltet die Therapie neben der gewünschten Wirkung auf das Ziel (-organ) auch unerwünschte Wirkungen an eigentlich funktionstüchtigen und z.T. lebensnotwendigen Körperzellen, Geweben oder Organen. Besonders beeinträchtigt werden z.B. die Knochenmarkszellen mit einer teilweise starken Funktionsstörung der Blutzellproduktion, der Magen-Darm-Bereich mit Erbrechen, Durchfall, Malabsorption; Entzündungen; Haarwachstumsstörungen bzw. Haarausfall, im intertriginösen (Körperfalten) Bereich auch Entzündungen der Haarfollikel mit Schweißdrüsenabszessen; Ulzerationen und Entzündungen der inneren und/oder äußeren Schleimhäute usw. Dosisabhängig und mit großen und damit schwer kalkulierbaren interindividuellen Unterschieden können auch Leber und Niere stark betroffen sein. Die beiden letztgenannten Organe sind nicht nur an der Entgiftung der therapeutisch eingesetzten Noxe beteiligt, sondern sind als physiologische "Blut- bzw. Körperentgifter" elementar wichtig und daher so weitestgehend wie möglich zu schonen.

Ein weiteres Problem ist ist das erhöhte Risiko für das Auftreten von sekundären und therapieassoziierten Sekundärtumoren (z.B. Lymphomen), wobei es bei der cytostatischen Therapie unbeabsichtigt auch zu einer irreversiblen Schädigung der genetischen Information einer gesunden Zelle gekommen ist. Das klinische Resultat kann eine unkontrollierte Vermehrung der geschädigten Zelle und damit eine weitere Tumorerkrankung sein, deren Entstehung zwar nicht vollständig von einem "natürlichen" Verlauf zu differenzieren ist ("Zufällige Häufung"), dessen Ursache aber mit hoher Wahrscheinlichkeit als therapieassoziiert anzusehen ist.

Bei bestimmten Chemotherapeutika steigt die Gefahr unerwünschter und eventuell für den Patienten schwerwiegender "unerwünschter Arzneimittelwirkung" (Nebenwirkungen, UW) sprunghaft an. Beispiele sind die Kardiotoxizität mit Herzinsuffizienz (bei steigender Einzel- bzw. Gesamtdosis) bei der Anwendung von Doxorubicin und Danorubicin, Lungenfibrose bei Bleomycin und Nitrosharnstoffderivaten. Eine UW kann den Patienten vital gefährden oder chronisch schädigen und damit seine Lebensqualität massiv einschränken. Durch das Überschreiten einer bisher empirisch definierten Obergrenze ("Gesamtdosis") nach recht unselektiver Wirkstoffapplikation und Belassung des ("Rest"-) Wirkstoffes im Organismus kann u. U. eine sinnvolle Weiterbehandlung gefährdet sein, obwohl der Tumor auf die Behandlung "angesprochen" hätte. Die hier vorliegende Erfindung versucht dies durch eine selektivere und besser steuerbare Wirkstoff-(ab-)gabe zu verbessern.

Ferner kann eine sorgfältige Dosiseinstellung unter Abwägen der therapeutischen Wirkungen einerseits und der toxischen Wirkungen andererseits angezeigt sein und ggf. eine Begrenzung auf niedrige Wirkstoffdosen erforderlich machen. Eine Verabreichung von Wirkstoffen in niedrigen Dosen kann jedoch dazu führen, dass die am Zielort im Organismus erforderliche Schwellenkonzentration zur Herbeiführung einer Wirkung nicht erreicht wird. Werden toxische Wirkstoffe in niedrigen Dosen. über einen längeren Zeitraum verabreicht, besteht ferner die Gefahr der körpereigenen Abwehr durch Resistenzbildung (Multi Drug Resistance, MDR), s. J. Robert: "Multidrug resistance in oncology: diagnostic and therapeutic approaches", Eur J Clin Invest 29(6) (1999 ), S. 536-545.

Das US-Patent 5 622 713 beschreibt ein Verfahren zum Enttoxifizieren eines Individuums, das von einer Überdosis einer chemischen Substanz, wie zum Beispiel eines Wirkstoffs leidet, durch Injektion einer Liposomen-haltigen Lösung, die auf einen unphysiologischen pH von ≤5,4 oder ≥9,4 eingestellt wird. Durch die Einstellung der Liposomenlösung auf einen sauren pH werden basische chemische Substanzen und durch Einstellung der Liposomenlösung auf einen basischen pH werden saure chemische Substanzen gebunden. Ziel ist es, die freie Wirkstoffkonzentration im Blut zu verringern, und vor allem eine Enttoxifizierung der chemischen Substanz durch Metabolisierung via Liposomen in der Leber zu bewirken. Eine Abtrennung von Toxinhaltigen Liposomen mittels Dialyse wird erwähnt und experimentell gemäß Beispiel 3 zur Messung der Kinetik des Ausflusses einer toxischen Substanz aus den Liposomen verwendet.

In der US 4 643 718 werden Antigen- oder Antikörpermodifizierte Liposomen in einer Ultrafiltrationseinheit 20 vorgelegt. Die modifizierten Liposomen reagieren mit der entsprechenden, im Blut vorliegenden immunologisch zu entfernenden Substanz zur Bildung eines Immunreaktionskomplexes, der anschließend in einem Filter 22 entfernt wird.

Die GB 2 232 984 A beschreibt die Verwendung von Chitosan-Kügelchen, an die Protein A oder Lectin kovalent gebunden sind, zum Binden von Immunglobulinen.

Die EP 0 361 894 A beschreibt eine spezielle Technik zur Beladung von Liposomen durch amphiphatische Moleküle sowie deren kontrollierte Freisetzung.

Die EP 0 120 445 A beschreibt die Entfernung hochmolekularer Spezies wie Lipoproteine, die für den Körper schädlich sein können, in einem extrakorporalen Kreislauf.

Die Aufgabe der vorliegenden Erfindung bestand somit darin, herkömmliche Therapiemöglichkeiten auf der Grundlage potentiell toxischer Wirkstoffe zu verbessern.

Die Aufgabe wird erfindungsgemäß durch eine Verwendung gemäß Anspruch 1 gelöst. Bevorzugte Weiterbildungen der erfindungsgemäßen Verwendung sind in den Unteransprüchen angegeben.

Nach Verabreichung eines therapeutischen Wirkstoffs bzw. einer den Wirkstoff enthaltenden pharmazeutischen Zusammensetzung in Form des partikulären Trägersystems können spezielle Eliminierungsverfahren durchgeführt werden. Dabei kann, wie nachfolgend beschrieben, die medizinische Therapie auf der Basis mikropartikulärer (Drug Delivery) Systeme erfolgen, wonach das eigentliche Eliminierungsverfahren durchgeführt wird.

Das erfindungsgemäße Konzept beruht darauf, dass potentiell toxische oder pathologische Wirkstoffe (im Folgenden häufig als toxische und/oder schädliche Stoffe bezeichnet), mittels Partikeln, die die toxischen bzw. schädlichen Stoffe binden, aufnehmen und/oder tragen können, indirekt durch eine auf die Partikel abzielende Abtrennung im extrakorporalen Entfernungsschritt oder wahlweise in der körperfremden Einrichtung effizient aus einem Organismus eliminiert werden können. Ein besonderer Vorteil der erfindungsgemäßen Verwendung besteht darin, dass toxische therapeutische Wirkstoffe bereits zur Therapie in Form geeigneter und.an sich bekannter partikulärer Trägersysteme appliziert, nach Erreichen des Wirkoptimums über den partikulären Träger aber aus einer geeigneten Körperflüssigkeit, insbesondere aus dem Blut, wieder entfernt werden können. Somit wird ein Synergismus im Sinne einer verminderten Toxizität erreicht, wobei sich eine verbesserte Verträglichkeit und verringerte Toxizität von herkömmlichen, ortsspezifischen bzw. Target-gerichteten Drug Delivery-Systemen auf der Basis einer Wirkstoff/Träger-Einheit mit der sehr effizienten Möglichkeit der Eliminierung solcher makroskopischer Wirkstoffträger in Kombination auswirkt. Außerdem werden durch das erfindungsgemäße Konzept die toxischen Stoffe dem natürlichen Clearance-Kreislauf bzw. der physiologischen Verstoffwechslung entzogen und somit die auf die natürliche Entgiftung spezialisierten Organe wie Leber, Galle, Niere usw. geschont.

Mit der erfindungsgemäßen Verwendung können alle potentiell toxischen und/oder schädlichen Wirkstoffe wieder aus dem Organismus entfernt werden, die bereits im Rahmen einer Therapie an die wahlweise einzusetzenden Partikel gebunden bzw. eingeschlossen waren. Gemäß der vorliegenden Erfindung ist es ferner möglich, die extrakorporale Eliminierung mit einer extrakorporalen Therapiegabe via entsprechender Körperflüssigkeiten wie Blut zu kombinieren. Im Extrakorporalkreislauf können zum Beispiel die Temperatur und/oder der pH-Wert so eingestellt werden, wie es im Rahmen gewünschter biochemischer Reaktionen oder Wechselwirkungen notwendig ist. Zum Verhalten von biologischen Membranen, z.B. von Liposomen, existiert reichhaltige Literatur, wobei in Bezug auf die Temperatur- bzw. pH-vermittelte Zufuhr auf zwei aktuelle Quellen direkt verwiesen wird, in denen einige klinische Anwendungsmöglichkeiten beschrieben werden: A. Hillery: "Heat-sensitive liposomes for tumour targeting", Drug Discov Today 6(5) (2001), S. 224-225; und I.M. Hafez IM et al.: "Tunable pH-sensitive liposomes composed of mixtures of cationic and anionic lipids", Biophys. J. 79(3) (2000), S. 1438-46. Im Rahmen der Wirkstoffbeladung von therapeutisch angewendeten und kommerziell erhältlichen Liposomen wird beispielsweise durch einen pH-Gradienten die Aufnahme des Wirkstoffes in den bereits formierten Partikel induziert (s. S.H. Hwang et al.: "High entrapment of insulin and bovine serum albumin into neutral and positively-charged liposomes by the remote loading method", Chem Pharm Bull (Tokyo) 48(3) (2000), S. 325-9; S.H. Hwang et al.: "Remote loading of diclofenac, insulin and fluorescein isothiocyanate labeled insulin into liposomes by pH and acetate gradient methods", Int J Pharm 179 (1-) (1999), S. 85-95; E. Maurer-Spurej et al.: "Factors influencing uptake and retention of amino-containing drugs in large unilamellar vesicles exhibiting transmembrane pH gradients", Biochim Biophys Acta 1416(1-2) (1999), S. 1-10; D.B. Fenske et al.: "Ionophore-mediated uptake of ciprofloxacin and vincristine into large unilamellar vesicles exhibiting transmembrane ion gradients", Biochim Biophys Acta 1414(1-2) (1998), S. 188-204; M. Gulati et al.: "Study of azathioprine encapsulation into liposomes", J Microencapsul. 15(4) (1998), S. 485-94). Dieses Prinzip kann nicht nur zur Beladung, sondern auch zur erfindungsgemäßen Elimination bzw. Reduktion endogener Stoffwechselprodukte oder exogen zugeführter Noxen (z.B. im Rahmen von Intoxikationen) sowohl intra- (in der körperfremden Einrichtung) als auch insbesondere extrakorporal angewandt werden. So kann z.B. eine Körperflüssigkeit wie Plasma *in vivo* oder *ex vivo* mit einer Liposomensuspension versetzt werden, um durch einen spezifischen oder unspezifischen Transport in die Liposomen unerwünschte Substanzen durch die zugesetzten Liposomen zu binden. In diesem Zusammenhang wird z.B. auf die US- Patent-Nrn. 5,843,474, 6,079,416, 5,858,400 (K.J. Williams) sowie 6,139,871 (Hope und Rodrigueza) verwiesen. Die Liposomen werden dann in einem anschließenden Schritt mit einem der unten beschriebenen Verfahren eliminiert.

Die toxischen Stoffe bzw. schädliche Substanzen schließen insbesondere solche therapeutische Stoffe ein wie z.B. herkömmliche pharmazeutische sowie rekombinante Wirkstoffe, DNAs und RNAs jeglicher Art wie für die Gentherapie oder die Antisensetechnologie einsetzbar, Radionuklide etc. Die im Rahmen der vorliegenden Erfindung gewünschte Entfernung des potentiell toxischen und/oder schädlichen Stoffs kann eine möglichst weitgehende Eliminierung, aber auch lediglich eine gewünschte Reduzierung des Stoffs bedeuten. Eine lediglich teilweise Reduzierung kommt z.B. dann in Frage, wenn die im Körper befindliche Dosis eines therapeutischen Wirkstoffs eingestellt oder einem gewünschten pharmakokinetischen Verlauf angepasst werden soll. Die zu dem jeweils zu entfernenden Stoff passende Partikelart kann vom Fachmann ohne weiteres, gegebenenfalls auch aus mehreren Möglichkeiten verschiedener Partikelarten, ausgewählt werden, wie nachfolgend näher beschrieben.

Als Partikel sind jegliche mikropartikulären Träger oder Transportvehikel geeignet, die die potentiell toxischen und/oder schädlichen Stoffe und insbesondere die therapeutischen Wirkstoffe binden, aufnehmen und/oder tragen können ausgewählt aus Liposomen, Mikrosphären, Nanopartikeln, Niosomen oder Polymerpartikeln. Die erfindungsgemäß eingesetzten und vor dem eigentlichen Eliminierungsschritt dem Körper applizierten Partikel sollten vorzugsweise eine hohe Bindungs- bzw. Trage-Kapazität in Bezug auf den toxischen Stoff haben, selbst nicht oder nur begrenzt toxisch und nicht immunogen sein und, wenn gewünscht, für die vorangehende Therapie eine selektive Wirkstoffzufuhr erlauben. Der makroskopische Partikelträger kann natürlichen oder künstlichen Ursprungs sein oder eine artifizielle Modifikation von natürlichen Vehikeln darstellen. Beispielsweise können mikropartikuläre Trägerteilchen eingesetzt werden, die aus herkömmlichen Drug Delivery-Systemen bekannt sind. Besonders geeignet sind Liposomen, Mikrosphären, Nanopartikel, Niosomen, Polymerpartikel, Lipoproteine, Viruspartikel (Viren, Virushüllen und andere, modifizierte Viruspartikel, deren Virulenzeigenschaft entfernt wurde oder in anderer Weise modifiziert wurde) und bestimmte Zellarten wie Blutzell-Subtypen, z.B. Erythrozyten und Lymphozyten.

Diese mikropartikulären Wirkstoffträgersysteme werden z.B. beschrieben durch P. Zanoviak, "Pharmaceutical Dosage Forms", insbesondere Kapitel 7.3, in "Ullmann's Encyclopedia of Industrial Chemistry", Vol. A 19, 5th ed., 1991, S. 241-271, mit weiteren Nachweisen wie folgt: E. Timlinson: "Site-Specific Drug Delivery" in G.S. Banker, C.T. Rhodes (Hrsg.): Modern Pharmaceuticals, 2. Auflage, Marcel Dekker, New York 1990, S. 673-694; S.N. Mills, S.S. Davis: "The Targeting of Drugs/Controlled Drug Delivery" in L. Illum, S.S. Davis (Hrsg.): Polymers in Controlled Drug Delivery, IOP Publishing, Bristol 1987, S. 4-6; P. Arthurson: "Site Specific Drug Delivery/The Fate of Microparticulate Drug Carriers after Intravenous Administration" in L. Illum, S.S. Davis (Hrsg.): Polymers in Controlled Drug Delivery, IOP Publishing, Bristol 1987, S. 15-24; R.L. Juliano, D. Layton: "Liposomes as a Drug Delivery System" in R. L. Juliano (Hrsg.): Drug Delivery Systems, Oxford University Press, New York 1980, S. 189-236; und R.C. Oppenheim: "Nanoparticles" in R.L. Juliano (Hrsg.): Drug Delivery Systems, Oxford University Press, New York 1980, S. 177-188. Weitere geeignete Wirkstoff/Träger-Konjugate werden beschrieben in:
J.P. Benoit et al.: "Les formes "vectorisées" ou a "distribution module", nouveaux systèmes d'administration des medicaments.", J. Pharm. Belg. 41 (1986): S. 819-829;
F. Emmen und G. Storm: "Liposomes in Treatment of Infectious Diseases", Pharm. Weekblad (Sci) 9 (1987): S. 162-171;
G. Gregoriadis, J. Senior und A. Trouet (Hrsg.): "Targeting of Drugs", Plenum Press, New York 1982;
R. Lawaczeck: "Liposomen als Zielgerichtete Pharmakaträger", Deutsche Apotheker-Zeitung 127 (1987) : 1771-1773;
in Bezug auf Radionuklid-Beladung, s. K. Kostarelos und D. Emfietzoglou: "Tissue dosimetry of liposome-radionuclide complexes for internal radiotherapy", Anticancer Res. 20 (5A) (2000), S. 3339-3345).

Die eingesetzten Partikel enthalten Wirkstoffe. Somit ist die erfindungsgemäße Verwendung besonders vorteilhaft dadurch anzuwenden, dass die zu entfernenden Partikel als Wirkstoffträger in einer vorangehenden medizinischen Therapie dienten. Sind die therapeutischen Wirkstoffe über Trägerpartikel in den Organismus gelangt, kann zu jedem gewünschten Zeitpunkt überschüssiger, d. h. nicht an eigenzelluläre Strukturen oder Organe des Organismus gebundener Wirkstoff eliminiert oder zumindest reduziert werden. Mit der Erfindung können somit auch Vergiftungen oder ähnliche Intoxikationen eliminiert werden. Ferner kann die wahlweise vollständige oder teilweise Eliminierung mittels der Entnahme von Partikeln mit daran gebundenen toxischen oder schädlichen Stoffen vorteilhaft zur Steuerung bzw. Einstellung des jeweiligen Stoffgehalts im Organsimus oder zur Verbesserung der Dosierung oder des zeitlichen Therapieschemas eines therapeutischen Wirkstoffs eingesetzt werden.

Je nach Zusammensetzung der verwendeten Lipide bzw. Proteine und dem Besatz mit Apolipoproteinen zeigen die Partikel unterschiedliche Bindungsaffinitäten, Halbwertszeiten, chemische Bindungen bzw. elektrostatische Wechselwirkungen mit Körpergeweben bzw. Zellen, sowohl gesunder als auch maligne transformierter Zellen (Rezeptorbesatz). Durch unterschiedlichen Besatz der Lipoproteinpartikel (natürliche oder artifizielle), der liposomalen Partikel und der genannten Trägersysteme kann eine erhöhte Spezifität bei der Bindung, Internalisation, Degradation und damit der (intra-) bzw. extrazellulären Freisetzung und Wirkung in bzw. an der Zelle erreicht werden. Die sogenannte Clearance (Aufnahme in Zellen bzw. Ausschleusung in Körperflüssigkeiten wie Galle, Urin etc.) sowie Verstoffwechslung (z.B. Abbau in Leber und / oder Niere) der Partikel sowie der transportierten Substanzen kann mit der vorliegenden Erfindung entsprechend beeinflußt oder modifiziert werden.

Als Beispiele können das rezeptorvermittelte DrugTargeting, genannt werden, die aufgrund unterschiedlicher Rezeptoren bzw. Oberflächenproteinen bzw. Markern (z.B. Glykoproteine, "Glykokalix") unterschiedliche Affinität zu Wirkstofftragenden oder "leeren" Partikeln haben.

Die Weitergabe an andere Körperflüssigkeiten.(z. B. aus dem Blut in den Urin) kann zu einer Konzentration der Partikel bzw. des Wirkstoffes führen. Diese Anreicherung kann therapeutisch gewünscht sein.

Die Bindung des Stoffs bzw. der Stoffe an die Partikel kann für die vorgefertigte therapeutische Form in jeglicher geeigneter Form erfolgen, wobei chemische Bindungen wie kovalente Bindungen, elektrostatische Wechselwirkungen, hydrophobe oder hydrophile Wechselwirkungen, spezifische Konjugatbildungen wie mittls Antikörpern bzw. Antikörperbestandteilen oder Rezeptorbindungen, der Einschluß in eine Partikelmembran oder in dessen wässriger oder lipophiler Phase, beziehungsweise eine rein physikalische Absorption oder Adsorption geeignet sind.

Als Beispiele für im Rahmen der Erfindung nutzbaren leeren, zur Aufnahme von toxischen oder anderweitig schädlichen Substanzen geeigneten Partikel sind die von K.J. Williams in den US Patenten Nr. 5,843,474, Nr. 6,079,416, Nr. 5,858,400 und die von M. Hope und W. Rodrigueza im US Patent Nr. 6,139,871 beschriebenen Liposomen-Systeme zu nennen. Die genannten Systeme beruhen jedoch auf einem anderen Prinzip: dort geht es um therapeutische Ansätze, um systemisch zu einer LDL-, Cholesterin- oder Plaquereduzierung beizutragen und dadurch Atherosklerose oder Nierenerkrankungen zu behandeln, ohne jedoch eine Eliminierung durch Entfernung der mit dem schädlichen Stoff beladenen Partikel aus einer Körperflüssigkeit in einem extrakorporalen Schritt oder in einer körperfremden Einrichtung in Betracht zu ziehen. Auf ähnliche Arbeiten wird ebenfalls verwiesen: K.J. Williams et al.: "Structural and metabolic consequences of liposomelipoprotein interactions", Adv. Drug Deliv. Rev. 32 (1-2) (1998), S. 31-43; K.J. Williams et al.: "Rapid restauration of normal endothelial functions in genetically hyperlipidemic mice by a synthetic mediator of reverse lipid transport", Arterioscler. Thromb. Vasc. Biol. 20 (4) (2000), S. 1033-1039; M. Aviram et al.: "Macrophage cholesterol removal by triglyceride-phospholipid emulsions", Biochem. Biophys. Res. Commun. 155 (2) (1988), S. 709-713. Andererseits werden gemäß der vorliegenden Erfindung die Konjugate, die sich nach der Aufnahme oder Bindung des toxisch bzw. schädlichen Wirkstoffs in oder an die Partikel binden, im anschließenden Entfernungsschritt eliminiert. Auch artifizielle Lipoproteine, wie z. B. von Barenholz, et al. (US Patent Nr. 5,948,756) und von Levine et al. (US Patent Nr. 5,128,318) beschrieben, sind einsetzbar und anschließend gemäß der vorliegenden Erfindung eliminierbar.

Ferner ist es bekannt, dass Erythrozyten zur Aufnahme und Verkapselung von Wirkstoffen *in vivo* eingesetzt werden können. So zeigt die Dissertation von Dr. Klaus Claußen (Dissertation der Fakultät für Naturwissenschaften der Martin-Luther-Universität Halle-Wittenberg, August 1989), dass Erythrozyten *in vivo* mit ausgewählten Östrogenen beladen werden können.

Eine Beladung kann wie oben beschrieben, insbesondere über chemische Affinitäten wie elektrostatische Wechselwirkungen oder hydrophobe Wechselwirkungen, oder über spezifische Konjugatsbildungen, z.B. über an die Partikel gebundene spezifische Antikörper gegen den toxischen Stoff oder durch andere Hilfsstoffe wie Rezeptoren oder Akzeptoren, erreicht werden. Hierfür können die oben genannten natürlichen, artifiziellen oder modifizierten Partikel, je nach Art des zu bindenden bzw. aufnehmenden Stoffs, in der Zusammensetzung varriert und wahlweise mit Bindungshilfsstoffen versehen werden.

Unter den erfindungsgemäß einzusetzenden Mikropartikeln sind Liposomen besonders bevorzugt. Liposomen sind gut charakterisiert und können in ihren Eigenschaften und Stoffzusammensetzungen im Sinne der vorliegenden Erfindung gut variiert werden. Liposomen sind nicht nur in einer Vielzahl verschiedener Zusammensetzungen und Strukturen bekannt und verfügbar und bereits in der Praxis für eine therapeutische Behandlung von Krankheiten über deren Wirkstoffbeladung einsetzbar. Es wird verwiesen auf D.D. Lasic: "Novel applications of liposomes", Trends Biotechnol. 16(7) (1998), S. 307-321; A. Chonn und P.R. Cullis: "Recent advances in liposomal drug-delivery systems", Curr Opin Biotechnol. 6(6) (1995), S. 698-708; sowie U. Massing: "Cancer Therapy with Liposomal Formulations of Anticancer Drugs", Symposium Pharmakokinetics and Onkology, S. 87, basierend auf einem Kongress der Society of Clinical Pharmacology and Therapy, Köln, 17. Oktober 1996. Im Rahmen der Erfindung wurde gefunden, dass diese Partikelart eine sehr effiziente Eliminierung im anschließenden Eliminierungsschritt der mit dem toxischen bzw. schädlichen Stoff beladenen Liposomen erlaubt.

Die Größe der erfindungsgemäß einzusetzenden Partikel kann je nach Partikeltyp verschieden sein und liegt im allgemeinen im Nanometerbereich bis hin zum Mikrometerbereich. Im Hinblick auf eine leichte Durchgängigkeit im vaskulären System liegt die mittlere Partikelgröße vorzugsweise im Bereich von 10 nm bis 10 µm. Für die Partikeltypen künstlichen Ursprungs wie Nanosphären, Nanopartikel, Polymerpartikel, artifizielle oder natürliche, ggf. künstlich modifizierte Lipoproteine und insbesondere Liposomen sind mittlere Größen mit durchschnittlichem Durchmesser von 10 bis 300, insbesondere von 40 bis 200 nm weiter bevorzugt, weil in diesem Bereich ein guter Kompromiss zwischen der Phagozytoseneigung gegenüber relativ großen Partikeln einerseits und der Beladungskapazität der Partikel andererseits erreicht wird. Beim Einsatz von Partikeln natürlichen Ursprungs kommen jedoch eher größere Partikelgrößen in Frage, in Übereinstimmung mit der natürlichen Größe.

Die oben beschriebenen und - nach erfolgter Applikation - die potentiell toxischen Stoffe wie Therapeutika oder Noxen tragenden bzw. damit beladenen Partikel werden im Rahmen des erfindungsgemäßen Verfahrens in einem extrakorporalen Schritt oder in einer körperfremden Einrichtung aus einer Körperflüssigkeit entfernt. Bei der erstgenannten Alternative, dem extrakorporalen Schritt, handelt es sich bei der Körperflüssigkeit geeigneterweise um eine zuvor aus dem Körper entnommene Körperflüssigkeit oder Gewebsflüssigkeit, insbesondere Blut oder Blutplasma. Bei der alternativen Vorgehensweise kann als körperfremde Einrichtung eine Einrichtung zur Entfernung der Partikel aus einer in der Körperhöhle befindlichen Flüssigkeit oder Gewebsflüssigkeit, wie aus Aszites, Pleuraerguss, Harnflüssigkeit, aus dem Peritonealbereich, aus Sekreten oder Ausscheidungssäften wie Speichel, Liquor, Gallenflüssigkeit, Lymphe, Pankreassekret usw., vorgesehen werden.

Die extrakorporale Entfernung der Partikel erfolgt am besten durch Blutentnahme mittels selektiver Blutreinigungsverfahren. Solche Blutreinigungsverfahren sind als solche bekannt und dem Fachmann geläufig und können auf die jeweils zu entfernenden Partikel je nach Partikelart und -charakteristika abgestimmt werden. Gegenüber herkömmlichen Blutreinigungsverfahren zur Entfernung toxischer oder pathogener Substanzen aus dem Blut besitzt die erfindungsgemäße, auf den Trägerpartikeln beruhende Verwendung jedoch deutliche Vorteile, weil Partikelträger-gebundene toxische Wirkstoffe gezielter und effizienter aus dem Blut entfernt werden können. Zudem wird im Fall eines vorangehenden therapeutischen Ansatzes durch die Erfindung gleichzeitig eine Kombination mit dem vorangehenden therapeutischen Konzept auf der Basis von auf Partikelträgern basierenden Drug Delivery-Systemen bewirkt. So wird erfindungsgemäß erreicht, dass nach dem Zeitpunkt des Wirkoptimums ohne weitere systemische Toxizität und unter Schonung des körpereigenen Clearance-Systems und der dazu gehörenden Zelltypen und Organe (Phagocytosesystem, Leber, Niere und Milz) der Partikel-gebundene toxische Stoff bzw. Wirkstoff aus dem Blut effizient und mit Hilfe technisch bereits hervorragend etablierter und klinisch an vielen medizinischen Zentren eingesetzten Verfahren eliminiert werden kann. Zur Beschreibung geeigneter Verfahren in der Literatur, s. B. R. Gordon und S. D. Saal: "Current status of low density lipoprotein-apheresis for the therapy of severe hyperlipidemia", Curr. Opin. Lipidol. 7 (1996), S. 381-384; K. Kajinami und H. Mabuchi: "Therapeutic effects of LDL apheresis in the prevention of atherosclerosis", Curr. Opin. Lipidol. 10. (1999), S. 401-406; N. Koga: "Efficacy and safety measures for low density lipoprotein apheresis treatment using dextran sulfate cellulose columns", Ther. Apher. 3 (1999), S. 155-160; R. S. Lees et al.: "Non-pharmacological lowering of low-density lipoprotein by apheresis and surgical techniques", Curr. Opin. Lipidol. 10 (1999), S. 575-579; G. R. Thompson und Y. Kitano: "The role of low density lipoprotein apheresis in the treatment of familial hypercholesterolemia", Ther. Apher. 1 (1997), S. 13-16; T. Yamamoto und T. Yamashita: "Low-density lipoprotein apheresis using the Liposorber system: features of the system and clinical benefits", Ther. Apher. 2 (1998), S. 25-30. Mit der erfindungsgemäßen Verwendung ist es sogar möglich, dass die die Wirkstoffe enthaltenden oder tragenden Partikel nach ihrer Entfernung aus der Körperflüssigkeit wiedergewonnen und einer erneuten therapeutischen Anwendung zugeführt werden können, wobei allerdings auf die notwendige Sterilität bzw. bakeriologische und infektiologische Unbedenklichkeit der wiedergewonnenen und erneut anzuwendenden Drug Delivery-Partikel zu achten ist.

Unter Berücksichtigung der Größe und der Art der jeweils eingesetzten, aus einer Körperflüssigkeit zu eliminierenden Partikel sind zu deren Entfernung vor allem Verfahrensschritte geeignet, die auf dem Prinzip der Fällung, der Filtration, der Chromatographie und/oder der Adsorption der Partikel beruhen. Es wurde gefunden, dass dabei z.B. auf herkömmliche Blut-Aphereseverfahren zurückgegriffen werden kann. Solche Aphereseverfahren sind z. B. bekannt zur selektiven Reduzierung des LDL-Gehalts im Blut zur Behandlung der Hypercholesterinämie. Diese in der Praxis bewährten Aphereseverfahren können sehr gut und einfach zur extrakorporalen Entfernung der mit den toxischen Stoffen bzw. Wirkstoffen beladenen Partikel genutzt werden. Die jährlich zehntausendfach (allein in Deutschland) eingesetzten verschiedenen Therapieverfahren der LDL-Apherese werden, mit gewissen Abweichungen je nach Verfahren, von den Patienten in aller Regel hervorragend toleriert. Hierzu gehören das so genannte HELP-System (siehe EP 0 174 478 A2; kommerziell genutzt von B.. Braun Melsungen, Deutschland), die Doppelfiltrationstechnik (Kaskadenfiltration) bzw. Membrandifferentialfiltration (MDF), das DALI-Verfahren zur direkten Adsorption aus Vollblut (s. K. Derfler und W. Drumel in Eur. J. Clin. Invest. 28 (1998): 1003-1005; L.J. Dräger et al. in Eur. J. Clin. Invest. 28 (1998): 994-1002; kommerziell genutzt von Fresenius AG, St. Wedel, Deutschland), die Adsorption aus Blutplasma an Dextransulfat (s. N. Koga: "Efficacy and safety measures for low density lipoprotein apheresis treatment using dextran sulfate cellulose columns", Ther. Apher. 3 (1999), S. 155-160; und T. Yamashita: "Low-density lipoprotein apheresis using the Liposorber system: features of the system and clinical benefits", Ther. Apher. 2 (1998), S. 25-30; kommerziell erhältlich als LIPOSORBER^{™} von Kaneka, Osaka, Japan) und die Immunadsorption. Einen Überblick über verschiedene Aphereseverfahren in der Hypercholesterinämie-Behandlung zeigen die oben angegebenen Literaturstellen und die Wiener Klinische Wochenschrift im Band 112/Heft 2 (2000), siehe Editorial auf S. 49-51. Erfolgt die Entfernung der Partikel nicht direkt aus dem Vollblut, ist eine Blutzellseparation z.B. über Filtration oder Zentrifugation der Blutzellen und anschließender Entfernung aus dem Blutplasma angezeigt.

Obgleich die Entfernung aus dem Blut bzw. Blutplasma am einfachsten und auf der Basis von bereits existierenden Verfahren möglich ist, kann die Entfernung der Partikel auch aus einer anderen, aus dem Körper entnommenen Körperflüssigkeit, z.B. Gewebsflüssigkeit, Aszites, Pleuraerguss, Harnflüssigkeit, Flüssigkeit aus dem Peritonealbereich, Sekreten oder Ausscheidungssäften wie Speichel, Liquor, Gallenflüssigkeit, Lymphe, Pankreassekret etc., erfolgen. Die von den Partikeln befreite Körperflüssigkeit bzw. deren Bestandteile können, wenn physiologisch oder therapeutisch gewünscht, wieder dem Körper zugeführt werden.

Die Entfernung der Partikel mittels Fällung erfolgt vorzugsweise mit einem polyvalent geladenen oder ionisierbaren Fällungsmittel, wie Polyanionen oder Polykationen, in Verbindung mit entsprechend entgegengesetzt geladenen oder ionisierbaren Partikeln oder Partikelbestandteilen. Bevorzugte Beispiele für Polyanionen als Fällungsmittel für positiv geladene oder ionisierbare Partikel sind bevorzugt Heparin, Dextransulfat und Phosphorwolframsäure. Als geeignete Alternativen kommen andere multivalente Polymere in Betracht, wie Polysulfat, Polysulfonat, Polyphosphat, Polycarboxylat, Poly(meth)acrylat, Polyvinylsulfat, -sulfonat und -phosphat, Polystyrolcarboxylat und -sulfonat, anionische Polysacharide wie Polygalacturonate, Hyaluronsäure, Keratinsulfat, anionische Cellulosederivate, Algininsäure usw., anionische Polypeptide wie Polymere oder Copolymere mit Glutaminsäure- und/oder Asparaginsäureeinheiten, die Copolymere der Einheiten der genannten Polymere und dergleichen. Geeignete Polykationen als Fällungsmittel für negativ geladene oder ionisierbare Partikel schließen basische Polysaccharide wie Dextrane, Amylose, Amylopektin oder andere Polysaccharide, die primäre, sekundäre oder tertiäre Aminogruppen oder Alkylammoniumgruppen aufweisen, Polypeptide oder deren Copolymere mit basischen Aminosäureeinheiten wie Lysin, Arginin, Ornithin, und polymere Amine oder N-Heterocyclen wie Polyvinylamine, Polyallylamine, Polyvinylalkylamine und Polyvinyltrialkylamoniumsalze, Polyvinylpyridine und deren quaternäre Salze, Poly(aminoalkyl)vinylalkohole, die Copolymere der Einheiten der genannten Polymere und dergleichen.

Die Fällung erfolgt geeigneter Weise in Anwesenheit zweiwertiger Kationen, wie Ca²⁺, Mn²⁺ und/oder Mg²⁺. Die Fällungsmittel und ggf. die Hilfsstoffe können nach der Plasma-Separierung durch exogene Zuführung, z.B. durch geeignete Lösungen oder Puffergemische, zugegeben werden. Durch eine geeignete, nachgeschaltete Einrichtung, z.B. einer Dialyseeinheit, einer Adsorptionssäule etc., können die Ausgangskonzentrationen wieder hergestellt werden. Auch unerwünschte Substanzen, die dem Reaktionsgemisch zugegeben wurden, können vor der Rückgabe an den Patienten wieder entfernt werden.

Der Einsatz von Polyanionen als Fällungsmittel kommt vor allem dann in Betracht, wenn die Partikel positiv geladene oder ionisierbare Gruppen wie primäre, sekundäre oder tertiäre Amine oder quaternäre Ammoniumgruppen tragen, wie dies z.B. beim Einsatz von Lipoproteinpartikeln durch den ApoB-Bestandteil des LDL-Lipoproteins und beim Einsatz von positiv geladenen oder aufladbaren Membranbestandteilen von Liposomen der Fall ist. Es gibt eine Vielzahl von positiv aufladbaren bzw, permanent positiv geladenen Liposomen, die zum Zweck der Erfindung eingesetzt werden können, wobei auf folgende Literatur verwiesen wird:
O. Zelphati und F.C. Szoka, "Mechanism of oligonucleotide release from cationic lipids", Proc. Natl. Acad. Sci. USA 93(21) (1996), S. 11493-11498. Die in US 6,020,526 beschriebenen, bekannten und kommerziell erhältlichen kationischen Lipide als Liposomenbestandteile schließen N[1-(2,3-Dioleoyloxy) propyl]-N,N,N-trimethyl-ammoniumchlorid ("DOTMA"), Dioleoylphosphatidylethanolamin ("DOPE"), 1,2Bis(oleoyloxy)-3,3-(trimethylammonia)propan ("DOTAP") bzw. deren strukturellen Modifikationen, allein oder in Kombination, sowie die in diesem Dokument vorgeschlagenen cationic lipids auf Amidbasis ein. Andere beschriebene kationische Lipidverbindungen schließen solche ein, bei denen z.B. Carboxyspermin mit einem oder mehreren Lipiden konjugiert wurden, z.B. 5-Carboxyspermylglycin-dioctaoleoylamid ("DOGS") and Dipalmitoyl-phosphatidyletanolamin 5-carboxyspermyl-amid ("IDPPES"), s. z.B. Behr et al., U.S. Pat. No. 5,171,678. Ferner wurden als Lipidbestanteile kationische Cholesterinderivate ("DC-Chol") beschreiben, die zusammen mit DOPE in Liposomen eingebracht wurden (s. Gao, X. und Huang, L., Biochim. Biophys. Res. Commun. 179 (1991), S. 280 ff.). Ferner gibt es Lipopolylysin, das durch Konjugation von Polylysin an DOPE hergestellt wird, s. Zhou, X. et al., Biochim. Biophys. Acta 1065 (1991), S. 8 ff. Weitere, für kationische Liposomen geeignete Lipidbestandteile sind im US-Patent 6,172,049 beschrieben.

Andererseits gibt es die Möglichkeit, anionische Lipidbestandteile zum Aufbau anionischer und mit kationischen Fällungsmitteln präzipitierbarer Liposomen zu verwenden, z.B. die im US-Patent 6,120,751 beschriebenen anionischen Lipide Phosphatidylsäure, Phosphatidylglycerol, Phosphatidylglycerol-Fettsäureester, Phosphatidylethanolaminanandamid, Phosphatidylethanolaminethanandamid, Phosphatidylserin, Phosphatidylinositol, Phosphatidylinositol-Fettsäureester, Cardiolipin, Phosphatidylethylenglycol, azidisches Lysolipid, Sulfolipid, Sulfatid, gesättigte oder ungesättigte freie Fettsäure wie Palmitinsäure, Stearinsäure, Aarachidonsäure, Oleinsäure, Linolensäure, Linolinsäure und Myristinsäure.

Weitere geladene oder ionisierbare Lipidbestandteile zum Aufbau entsprechen geladener Liposomen sind kommerziell verfügbar von der Firma Avanti Polar Lipids, Inc., Alabaster AL 35007 (www.avantilipids.com).

In den eingesetzten Liposomen können zusätzlich zu den Lipidbestandteile weitere Bestandteile und Hilfsmittel eingebaut werden. Dies sind z.B. die oben erwähnten Substanzen zum Zweck einer spezifischen Anbindung (für die zielgerichtete Therapie und/oder die spezifische Eliminierung), Liposomenstabilisatoren wie Polyethylenglycol (PEG) oder andere Funktionsbestandteile und Polymere. So können zum Beispiel solche, von den Lipiden verschiedenen Bestandteile die Fällung oder andere Eliminierungstechniken unterstützen. Andere Polymerbestandteile als PEG sind z.B. Polyvinylpyrrolidon, Polyvinylalcohol, Polypropylenglycol, Polyvinylalkylether, Polyacrylamid, Polyalkyloxazolin, Polyhydroxyalkyloxazolin, Polyphosphazin, Polyoxazolidin, Polyaspartamid, ein Polymer der Sialinsäure, Polyhydroxyalkyl(meth)acrylat und Poly(hydroxyalkylcarboxylsäure).

Es hat sich gezeigt, dass die Bedingungen und Schritte, die im herkömmlichen HELP-Verfahren zur Eliminierung von LDL-Partikeln üblich sind (Heparin in zweiwertige Kationen enthaltenden, sauren Puffern im pH-Bereich von 4,8 - 5,4) nicht nur für die Heparin-induzierte Fällung von LDL, sondern auch auf Liposomen anwendbar ist, die positiv geladene oder aufladbare Gruppen enthielten, wie Phosphoglyceride mit Phosphatidylcholin- und Phosphatidylethanolamin-Einheiten.

Ferner ist die Membran-Differentialfiltration (MDF) bzw. Kaskadenfiltration gut geeignet. Aufgrund der Ausnutzung von mindestens zwei Filtersystemen mit einer vorläufigen Abtrennung von Blutzellen und Plasma gefolgt von weiteren Filtrationstrennungen der im Verfahren eingesetzten mikropartikulären Bestandteile von anderen Plasmabestandteilen ist eine effektivere Eliminierung der eingesetzten Partikel möglich. Dabei können die einzelnen Filtersysteme unter Beachtung der Größe der einzelnen Blutbestandteile und insbesondere der zu eliminierenden Partikel und der dadurch entsprechenden Porengröße der Filtermaterialien eingestellt bzw. ausgewählt werden.

Eine effiziente Eliminierung der Partikel ist ferner durch verschiedene Adsorptionsverfahren möglich, die nachfolgend näher beschrieben werden.

Eine Möglichkeit auf der Grundlage der Adsorptionstechnik beruht darauf, dass die Adsorption über elektrostatische Wechselwirkungen zwischen den Partikeln, die über geladene Gruppen verfügen oder ionisierbar sind, und dem entsprechend entgegengesetzt geladenen oder ionisierbaren Adsorbensmaterial erfolgt. Geeignet für ein solches Adsorbensprinzip sind polykationische oder polyanionische Adsorbensmaterialien zur Eliminierung der entsprechend entgegengesetzt negativ bzw. positiv geladenen oder aufladbare Gruppen tragenden Partikel. Als Beispiele sei verwiesen auf die oben bereits im Zusammenhang mit der Fällung beschriebenen Polyanionen oder Polykationen, insbesondere auf die gegenüber positiv geladenen oder aufladbaren LDL- oder Liposomen-Partikel gut anzuwendenden Polyanionen wie Polyacrylamid oder Dextransulfat. Die Polykationen oder Polyanionen können als Liganden kovalent an entsprechende Adsorptions-Trägermaterialien wie Polyacrylamid, Sephadex^{™}, etc. gebunden sein. Als besonders geeignet und effizient ist in diesem Zusammenhang das bereits genannte DALI-Aphereseverfahren zu erwähnen (Lit. s. oben). Obgleich bisher lediglich zur Behandlung der Hypercholsterinämie bekannt, kann dieses Verfahren auch zur Eliminierung anderer, erfindungsgemäßer Trägerpartikel toxischer Stoffe bzw. Wirkstoffe als von LDL-Partikeln, z.B. bei Liposomen, angewandt werden. Der Hauptvorteil liegt darin, dass der Reinigungsschritt aus dem Vollblut erfolgen kann. Das DALI-Prinzip beruht auf einer Kombination aus Adsorption und Größenausschluss-Chromatographie (Gelchromatographie). Hierzu sind die kleinen Adsorber-Kügelchen (beads) mit mittlerem Durchmesser von 150 - 200 µm porös ausgestaltet und an den äußeren und inneren (Poren-) Oberflächen mit Polyanionen, speziell Polyacrylat, als Adsorbensmaterial behaftet.

Als eine spezielle Art der Adsorptionstechnik ist ferner die Immunadsorption als Möglichkeit der Partikelabtrennung zu nennen. So können Antikörper, die spezifisch mit Komponenten bzw. Oberflächenbestandteilen der erfindungsgemäß eingesetzten Mikropartikel interagieren, an einen Adsorbensträger kovalent gebunden werden und somit zur selektiven Entfernung der Mikropartikel aus der entnommenen Körperflüssigkeit oder einem nach Zwischenseparationsschritten erhaltenen Folgeprodukt dienen. Auf dieser Grundlage lassen sich wiederum hervorragend die Konzepte der Target-spezifischen Therapie in Kombination mit einer selektiven Eliminierung nach einem gewissen Zeitintervall der Gabe entsprechender Drug Delivery-Systeme verwirklichen. Die Selektivität für die zielgerichtete Therapie einerseits und die Erkennung durch die an das Adsorbensmaterial gebundenen Antikörper andererseits erfolgt geeigneter Weise über ein bestimmtes Strukturmerkmal ("struktureller Code"). So kann z.B. ein an den Mikropartikeln gebundener, Carzinom-spezifischer Antikörper für die Selektivität der ortsspezifischen Therapie sorgen und gleichzeitig für den anschließenden Eliminierungsschritt als Epitopen tragendes Antigen zur Erkennung - mittels eines Gegen-Antikörpergebundenen Immunadsorbensmaterials - dienen. Als ein weiteres zielgerichtetes Drug Delivery-System mit kombiniertem, extrakorporalem Eliminierungsschritt kommen spezialisierte Zellen wie cytotoxische T-Zellen und NK-Zellen (*Natural Killer Cells*) in Frage, die nach der Therapie über die zellspezifischen Antigene mittels Immunadsorption wieder aus dem Blutkreislauf eliminiert werden können. Nach einem solchen Prinzip sind auch die bereits beschriebenen Viren und modifizierten Viren einsetzbar und eliminierbar.

In Fällen, bei denen die toxische Stoffe aufnehmenden Mikropartikel eine relativ geringe Größe im Nano- und Subnanometerbereich aufweisen, sind ferner als spezifische Entfernungsverfahren auch chromatographische Verfahren anwendbar. In solchen Fällen sind vorgeschaltete, grobe Trennschritte wie Plasmaseparierung angebracht. Das chromatographische Prinzip kann dabei auf einer Abtrennung auf der Basis der Größenunterschiede, auf der Basis einer elektrostatischen Bindung an den Festphasenträger (Ionenaustausch-Chromatographie) und/oder auf der Basis einer adsorptiven Bindung an hydrophobe Festphasenträger oder über eine Antigen/Antikörper-Wechselwirkung mit einem spezifischen Festphasenträger erfolgen. Beispielsweise beschreiben E. Choice et al. in Anal. Biochem. 270 (1999), S. 1-8, und J. Turanek in Anal. Biochem. 218 (1994), S. 352-357, die chromatographische Gewinnung von Liposomen mittels FPLC. Bei diesen Arbeiten geht es jedoch nicht um einen therapeutischen Ansatz, geschweige denn um eine bewusste oder substanzielle Eliminierung von mit toxischen Stoffen beladenen Liposomen.

Vielmehr beschreibt Choice et al. die FPLC-Methode lediglich als analytische Möglichkeit, die Liposomenstabilität im Blut zu untersuchen und liposomale Drug-Carrier Systeme zu charakterisieren, während J. Turanek die FPLC-Methode zur Liposomenherstellung durch die Extrusionstechnik nutzt.

Es ist zu betonen, dass die oben beschriebenen Verfahren bzw. Schritte zum Entfernen der Partikel aus der entnommenen Körperflüssigkeit oder in der körperfremden Einrichtung jeweils einzeln oder in Kombination miteinander oder mit anderen herkömmlichen Blutreinigungsverfahren durchgeführt werden können. Geeigneterweise erfolgt die Eliminierung in einem Kreislauf, bei dem der Eliminierungsschritt zwischen der Entnahme und der nach der Eliminierung erfolgenden Rückführung der Körperflüssigkeit erfolgt. Somit können,nach dem Eliminierungsschritt soweit erwünscht Bestandteile der Körperflüssigkeit dem Körper wieder zugeführt werden. Ein solches Verfahren kann ohne weiteres in gut etablierte Dialyse- oder Aphereseverfahren eingebunden werden.

Im Rahmen einer vorgeschalteten Therapie mit mikropartikulären Drug Delivery-Systemen sind grundsätzlich jegliche therapeutischen Wirkstoffe zum Binden an oder zum Einschluss in die Partikel geeignet. Die Anwendung des erfindungsgemäß verwendeten Eliminierungssystems erlaubt die Entwicklung einer völlig neuartigen Pharmakologie für bereits bekannte Wirkstoffe. Dies ist insbesondere in solchen Bereichen von höchstem Interesse, wo hochtoxische, stark schädigende oder für den Organismus kritische Wirkstoffe zum Einsatz kommen. In diesem Sinne ist die erfindungsgemäße Verwendung in Kombination mit einer vorangehenden medizinischen Therapie besonders dann sinnvoll, wenn die therapeutischen Wirkstoffe aus der Gruppe ausgewählt sind, die aus Cytostatika, Antibiotika, Antivirusmitteln, Antimykotika, gentherapeutischen Substanzen (also alle im Rahmen sogenannter Gentherapien eingesetzter Wirkstoffe wie Oligonukleotide, Ribozyme, DNAs, Plasmide, Vektoren und Liposomen- oder Viruspartikel-basierende Genfähren), Antikörpern, Cytokinen, Interferonen und Radionukliden besteht. Zu den Cytostatika zählen z.B. die bereits in liposomaler Form vorliegenden Wirkstoffe Daunorubicin bzw. Doxorubicin, ferner N-Lost Derivate, Ethylenimine, Alkylsulfonate, Nitrosoharnstoff-Derivate, Platinkomplexverbindungen, Dacarbacin und Procarbacin, Mitomycin, Altretamin, interkalierend wirkende Zytostatika, wie Actinomycine, Anthrazykline, Mithramycin, Amsacrin, Mitoxantron, Antimetabolite wie Folsäureantagonisten, Pyrimidinantagonisten, Purinantagonisten, Spindelgifte wie Vinca-Alkaloide und Podophyllotoxin-Derivate sowie Bleomycine, Hydroxyharnstoff, Mopidamol, L-Asparaginase und Interferone usw. Die Reihe dieser und anderer Wirkstoffklassen ist unbegrenzt. Neben den obigen-allgemeinen Literaturangaben zum mikropartikulären Drug Delivery seien an dieser Stelle insbesondere auf die Artikel von D.D.Lasic (1998), a.a.O., und A. Chonn und P:R. Cullis (1995), a.a.O., und für den Bereich der Liposomenvermittelten Radiotherapie auf die oben zitierte Quelle von K. Kostarelos und D. Emfietzoglou Bezug genommen. Ferner ist der therapeutische Ansatz in Kombination mit einer ggf. teilweisen Eliminierung des therapeutischen Agens besonders sinnvoll bei den problematischen gentherapeutischen Ansätzen, bei denen Oligonukleotide wie Antisensemittel oder Rybozyme, DNA-Plasmide, Vektoren oder ähnliche Genfähren auf Viren- oder Liposomenbasis zum Eisatz kommen, s. beispielsweise E. M. Hersh und A. T. Stopeck: "Advances in the biological therapy and gene therapy of malignant disease", Clin. Cancer Res. 3 (1997), S. 2623-2629; R. R. Weichselbaum und D. Kufe: "Gene therapy of cancer", Lancet 349 Suppl 2 (1997), SII10-SII12; P. J. Woll und I. R. Hart: "Gene therapy for lung cancer", Ann. Oncol. 6 Suppl 1 (1995), 73-77; sowie J.M. Brown und A.J. Giaccia "The unique physiology of solid tumors: opportunities (and problems) for cancer therapy", Cancer Res. 58(7) (1998), S. 1408-16.

Die Wirkstoffe können synthetischen, natürlichen oder semisynthetischen Ursprungs sein oder mikrobiologisch oder gentechnologisch hergestellt sein. Art und Herstellung der solche Wirkstoffe enthaltenden, mikropartikulären Drug Delivery-Systeme sind aus der oben genannten Literatur ersichtlich.

Im Rahmen der vorliegende Erfindung ist ferner ein Kit zur medizinischen Therapie Verwendbar, der
a) eine Präparation von Partikeln, die zum Binden, Aufnehmen und/oder Tragen von potentiell toxischen oder schädlichen Wirkstoffen in der Lage sind, und
b) Mittel zum Entfernen der Partikel aus Körperflüssigkeiten umfasst.

Zu diesem Aspekt kann in Bezug auf die die potentiell toxischen oder schädlichen Stoffe bindenden, aufnehmenden bzw. tragenden Partikel auf die obige Beschreibung verwiesen werden, insbesondere auf die Wahl bzw. die verschiedenen Ausgestältungsmöglichkeiten in Bezug auf Liposomen, selektiv entfernbare Zelltypen, Lipoproteine und Polymerpartikel. Der Kit ist besonders nützlich, weil es sich bei den toxischen bzw. schädlichen Stoffen um therapeutische Wirkstoffe handelt. Dabei können die in dem Kit vorliegenden Partikel bereits die therapeutischen Wirkstoffe einschließen, wenn eine ausreichende Stabilität herrscht. Wahlweise können auch die Partikelproben und die Probe mit dem therapeutischen Wirkstoff getrennt im Kit vorliegen und vor dem direkten Gebrauch zur Beladung der Partikel mit dem Wirkstoff zusammengegeben werden. Ein Beispiel für den letztgenannten Fall ist die Therapie-Kombination von Liposomen mit Doxorubicin, das unter dem Namen "TLC D-99" der Firma TLC vertrieben wird. Hier wird erst direkt vor der Injektion eine vom Anwenderpersonal vorgenommene Beladung der Liposomen mit dem Wirkstoff vorgenommen.

In Bezug auf die therapeutischen Wirkstoffe wird ebenfalls auf die obigen Ausführungen dazu verwiesen.

Die Mittel zum Entfernen der Partikel aus den Körperflüssigkeiten wie Blut, Blutplasma, Gewebsflüssigkeiten wie Aszites oder Pleuraerguss, Sekreten oder Ausscheidungssäften wie Speichel, Liquor, Gallenflüssigkeit, Lymphe, Pankreassekret, Harnflüssigkeit, Flüssigkeit aus dem Peritonealbereich etc. ergeben sich aus der Wahl der oben beschriebenen Reinigungsverfahren und entsprechen somit den technischen Mitteln, die zur Durchführung des jeweiligen Verfahrens geeignet bzw. üblich sind. Somit schließen die im Kit bereitgestellten Mittel jeweils geeignete Fällungsmittel, Kaskadenfiltrationsmittel, Adsorptionsmittel und/oder Chromatographiemittel zusammen mit den jeweils erforderlichen bzw. üblichen Reagentien wie Puffer, Waschlösungen oder weiteren Hilfsstoffen einzeln oder in Kombination ein.

Aufgrund der Tatsache, dass Liposomen in der Praxis bereits weit verbreitet sind und in einer Vielzahl unterschiedlicher Typen mit variablen Charakteristika zur Verfügung stehen, wird erfindungsgemäß in einem weiteren Gegenstand die Anwendung solcher Liposomen zur Eliminierung toxischer Substanzen aus dem menschlichen Körper zur Verfügung gestellt. Eine sehr nützliche Anwendung besteht darin, dass ein therapeutischer Wirkstoff die toxische Substanz darstellt und die mit diesem Wirkstoff beladenen Liposomen zur Therapie gegen Krankheiten eingesetzt wurden, bevor zur Vermeidung weiterer toxischer Wirkungen die Wirkstoffe wieder aus dem menschlichen Körper eliminiert wurden. In der Regel wird ein solches Therapiekonzept so ablaufen, dass die Wirkstoff-beladenen Liposomen auf herkömmliche Art, heute meist intravenös, in der Zukunft ggf. auch oral oder auf anderem Applikationsweg, appliziert werden und nach einem je nach Wunsch oder Bedarf gewählten Zeitintervall zumindest teilweise wieder aus dem Körper entfernt werden. Die Entfernung der Liposomen kann dabei nach irgendeinem der oben beschriebenen Eliminierungsverfahren erfolgen.

Somit stellt die Erfindung eine sehr effiziente Möglichkeit zur Entfernung von Wirkstoffen aus Körperflüssigkeiten wie Blut zur Verfügung. Über die im Rahmen der Erfindung zur Anwendung kommenden Mikropartikel können die therapeutischen Wirkstoffe nach Erreichen des Wirkungsoptimums bei trägergebundenen Drug Delivery-Systems, mit Hilfe von Verfahren, die auf die Entfernung partikulärer Bestandteile aus Körperflüssigkeiten wie Blut bzw. Blutplasma ausgerichtet sind, mit verhältnismäßig geringem Aufwand entfernt werden. Dabei kann auf herkömmliche und in der Praxis eingeführte Blutwäsche-/Blutreinigungs- bzw. Apherese-Verfahren und herkömmliche, partikuläre Wirkstoffträger-Konjugate zurückgegriffen werden. Aufgrund der Anwendung des erfindungsgemäßen Konzepts können hochtoxische Wirkstoffe selbst dann zum Einsatz kommen, wenn diese bisher aufgrund der toxischen Nebenwirkungen nicht oder nur in Extremfällen inzidiert waren. Darüber hinaus erlaubt das erfindungsgemäße System eine völlig neuartige Pharmakologie für bereits bekannte Wirkstoffe. So kann nach hochdosierten Applikationsgaben und der Anreicherung der Wirkstoff/Partikel-Konjugate am gewünschten Zielort, z.B: einem Tumor, der im Blut befindliche Dosisspiegel wieder reduziert werden. Die Anwendung des erfindungsgemäßen Konzepts in Verbindung mit einer durch verminderte Toxizität gekennzeichneten, modifizierten Pharmakologie verspricht somit eine höhere Wirksamkeit sowie eine verringerte Gefahr der körpereigenen Resistenzbildung gegen Wirkstoffe (Multi Drug Resistance). Der Organismus wird durch Anwendung des erfindungsgemäßen Verwendung ferner dadurch geschont, dass die entgifteten Körperflüssigkeiten, insbesondere die abgetrennten Blutbestandteile, dem Körper im Rahmen eines Kreislaufes wieder zurückgeführt werden können, wie dies in herkömmlichen Blutwasch- bzw. Aphereseverfahren üblich ist.

Die Erfindung wird nachfolgend anhand von nicht einschränkend zu verstehenden Beispielen näher erläutert.

### Beispiele

Es werden folgende Abkürzungen verwendet:
- DPPC: 1,2-O-Dipalmitoyl-sn-glycero-3-phosphocholin
- DPDAP: 1,2-O-Dipalmitoyl-3-dimethylammonium-propan
- DPTAP: 1,2-O-Dipalmitoyl-3-trimethylammonium-propan
- DSPE: 1,2-O-Distearoyl-sn-glycero-3-phosphoethanolamin
- DSPG: 1,2-O-Distearoyl-sn-glycero-3-phospho-rac-(1-glycerol)
- EGTA: Dinatriumsalz der [Bis-(aminoethyl)-glycolether]-N,N,N',N'-tetraessigsäure
- HEPES: 2-[4-(2-Hydroxyethyl)-1-piperazinyl-]-propansulfonsäure
- PC: Phosphatidylcholin
- POPC: 1-O-Palmitoyl-2-O-oleoyl-sn-glycero-1-phosphocholin
- Rpm: Umdrehungen pro Minute
- v/v: Volumen pro Volumen

### 1. Allgemeine Beschreibung der Materialien und Methoden

### 1.1 Radioaktive Substanzen

[1,2-³H]Cholesterylhexadecylether (spezifische Radioaktivität: 1,9 TBq/mMol) wurde von NEN Life Science (Frankfurt, Deutschland) geliefert.

### 1.2 Nichtradioaktive Substanzen

Von folgenden Firmen wurden die aufgeführten Chemikalien bezogen:
Avanti Polar Lipids Inc. (Alabaster, Alabama, USA): DPTAP, DPDAP
Pharmacia LKB Biotechnology (Uppsala, Schweden) Heparin-Sepharose
Sigma-Aldrich Chemie GmbH (Deisenhofen, Deutschland): Cholesterol
Sygena Ltd. (Liestal, Schweiz): DSPE, DPPC, DSPG, POPC Zinsser Analytik GmbH (Frankfurt, Deutschland): Biolute-S®
Alle übrigen Substanzen wurden im jeweils höchstmöglichen Reinheitsgrad von den üblichen Herstellern bezogen.

### 1.3 Herstellung von Pufferlösungen

Zur Herstellung von Pufferlösungen wurde Wasser aus einem Wasseraufbereitungssystem Milli-Q UF (Millipore GmbH, Eschborn, Deutschland) mit einem Widerstand von mindestens 18 MW·cm verwandt.

### 1.4 UV/VIS-Spektroskopie

Spektroskopische Messungen im UV-Bereich sowie im Bereich sichtbarer Wellenlängen wurden mittels eines UV/VIS-Spektrophotometers DU 600 (Beckman Instruments Inc., Fullerton, USA) durchgeführt. Je nach Versuchsführung wurden 0,5- oder 1-cm-Quarzküvetten benutzt.

### 1.5 Messung von Radioaktivität

Die Radioaktivität in flüssigen Proben wurde durch Flüssigkeitsszintillationsmessung (Wallac 1411, Berthold, Wildbad, Deutschland) bestimmt. Zur Ermittlung von absoluten Radioaktivitätsmengen wurde die Quenchkorrektur und ein externer Standard benutzt. Zur Messung der Radioaktivität in den Proben wurden maximal 1 ml der Probenlösung mit 10 ml Ultima Gold® (*Pakkard*) versetzt. Die Radioaktivität wurde frühestens nach 4 h bestimmt.

### 2. Herstellung und Charakterisierung von Liposomen

### 2.1 Herstellung von Liposomen

Die Herstellung von Liposomen erfolgte in einer modifizierten Form nach der Extrusionsmethode von MacDonald et al.: "Smallvolume extrusion apparatus for preparation of large, unilamellar vesicles", in Biochim. Biophys. Acta. 1061 (1991), S. 297-303.
Die einzelnen Lipide, die in der Liposomenmembran enthalten sein sollten, wurden in definierter Konzentration in Chloroform (POPC, DPPC, Cholesterol) oder in einem Gemisch aus Chloroform und Methanol im Verhältnis 2/1 (v/v) (DSPG, DSPE, DPDAP, DPTAP) gelöst. Diese Stammlösungen wurden bei - 20 °C gelagert. Zur Herstellung von Liposomen gewünschter Lipidzusammensetzung wurden die entsprechenden Aliquoten der Lipidstammlösungen in einen 25-ml-Rundkolben überführt. Die Lipidzusammensetzung der verwendeten Liposomen in Mol% ist in Tab. 1 angegeben.

**Tab. 1. Lipidzusammensetzung der verwendeten Liposomen in Molprozent**

| Lipidkomponente | DSPG-tragende Liposomen | Kontrollliposomen | DSPE-tragende Liposomen | DPDAP-tragende Liposomen | DPTAPtragende Liposomen |
|---|---|---|---|---|---|
| DPPC | 25 | 40 | 35 | 35 | 35 |
| POPC | 25 | 30 | 30 | 30 | 30 |
| Cholesterol | 40 | 30 | 30 | 30 | 30 |
| DSPG | 10 | - | - | - | - |
| DSPE | - | - | 5 | - | - |
| DPDAP | - | - | - | 5 | - |
| DPTAP | - | - | - | - | 5 |
| Nettoladung | negativ | neutral | neutral | neutral/positiv | positiv |

Zur radioaktiven Markierung der Liposomen wurden 0,25 µCi/mg Lipid (9,25kBq/mg) [³H]Cholesterylhexadecylether zu den Ansätzen gegeben.
Die resultierende Lipidmischung wurde im Vakuum vom Lösungsmittel befreit und anschließend 45 min lang im Ölpumpenvakuum getrocknet. Für die Liposomenpräparation wurde folgender Puffer (pH 7,4) verwendet (Liposomenpuffer) :

| | | |
|---|---|---|
| NaCl | 118 | mM |
| KCl | 4,74 | mM |
| KH₂PO₄ | 0,59 | mM |
| Na₂HPO₄ | 0,59 | mM |
| HEPES | 10 | mM |
| NaHCO₄ | 15 | mM |
| MgCl₂ | 1,18 | mM |

Vor seiner Verwendung wurde der Liposomenpuffer durch eine Membran mit einem Porendurchmesser von 220 µm filtriert. Um die Lipide zu resuspendieren wurde die gewünschte Menge an Liposomenpuffer zur getrockneten Lipidmischung gegeben und es wurde 15 min lang bei 40 °C und 50 Rpm mit Hilfe eines Rotationsverdampfers gerührt. Durch die Zugabe von 10-20 Glasperlen (710-1180 micron, Sigma) konnte das Resuspendieren der Lipide erheblich beschleunigt werden. Die zugegebene Menge an Liposomenpuffer wurde so gewählt, dass die erhaltene Suspension 10 - 40 mg Lipid/ml enthielt. Die Lipidsuspension wurde 30 s bei 1000 × g zentrifugiert und danach 2 h lang bei Raumtemperatur ruhen gelassen. Schließlich wurden die entstandenen multilamellaren Vesikel mit Hilfe einer Extrusionsapparatur LiposoFast® (Milsch-Equipment, Laudenbach, Deutschland) 12 mal durch eine Polycarbonatmembran (LFM-200, Milsch-Equipment) mit einem Porendurchmesser von 200 nm gedrückt und anschließend 21mal durch zwei übereinanderliegende Polycarbonatmembranen (LFM-100, Milsch-Equipment) mit einem Porendurchmesser von 100 nm gedrückt. Die Liposomen wurden bei 4 °C gelagert und nicht länger als 7 Tage nach ihrer Herstellung benutzt.

### 2.2 Charakterisierung der Liposomen

### 2.2.1 Größenbestimmung

Die Größe der Liposomen wurde mit einem ZetaSizer III (Malvern, England) mit Hilfe der Photonenkorrelatiosspektroskopie bestimmt (Washington, C. (1992) "Particle size analysis in pharmaceutics and other industries", (Rubinstein, M. H., ed.) Ellis Horwood, Ltd., London, England).Die Messung erfolgte in 3-ml-Einwegplastikküvetten, die etwa 0.3 mMole liposomales PC pro ml Zellpuffer enthielten. Jede Probe wurde dreimal vermessen und aus den drei Messwerten wurde der Mittelwert gebildet.

### 2.2.2 Bestimmung der Phospholipidkonzentration

Die Konzentration an cholinhaltigen Phospholipiden in den Liposomensuspensionen wurde auf photometrischem Wege bestimmt (Stewart, J. C. M. in "Colorometric Determination of Phospholipids with Ammonium Ferrothiocyanate", Anal. Biochem. 104 (1979), S. 10-14). Hierzu wurde eine Ammoniumferrothiocyanatlösung, bestehend aus 27.03 g FeCl₃•6H₂O und 30.04 g NH₄SCN, die in 1 l H₂O dest. gelöst wurden, hergestellt. Zur Durchführung der Phospholipidbestimmung wurde die zu untersuchende Liposomensuspension auf eine Gesamtlipidmenge von ca. 2 mg/ml Liposomenpuffer verdünnt. In einem Reaktionsgefäss wurden 1 ml CHCl₃ mit 750 µl der oben beschriebenen Ammoniumferrothiocyanatlösung überschichtet, und es wurden 25 µl der Liposomenverdünnung zugegeben. Die beiden Phasen wurden gründlich gemischt und anschließend durch fünfminütige Zentrigugation bei 2500 × g separiert. Nach der Zentrifugation wurde die obere wäßrige Phase entfernt. Die Chloroformphase wurde in eine 1-ml-Glasküvette überführt, und die Extinktion der Lösung wurde bei 485 nm in einem Photometer bestimmt. Als Blindwert diente eine analog behandelte Probe ohne Liposomen. Von jeder Liposomensuspension wurden 5 Proben vermessen und der Mittelwert aus diesen Proben berechnet.
Zur Eichung des Testes wurde eine Phospholipidstammlösung in CHCl₃ hergestellt. Die Eichung erfolgte durch jeweils 6 verschiedene Konzentrationen an POPC und DPPC im Bereich von 0 - 150 nmole PC/ml CHCL₃. In diesem Bereich ist der Test linear.

### 3. Bezugsbeispiel 1 - Kaskadenfiltration von Liposomen

Bei der Kaskadenfiltration zur Separation von Lipoproteinen aus dem Blut werden Membranen verschiedenen Porendurchmessern verwendet. Nach Plasmaseparation erfolgt die Abtrennung der LDL-Partikel hierbei gemäß Herstellerangabe durch eine Membran mit einem Porendurchmesser von 22 nm. Um herauszufinden, ob das Prinzip der Kaskadenfiltration auch auf Liposomen anwendbar ist, wurde die Filtrierbarkeit von Liposomen exemplarisch an zwei aufeinanderfolgenden Filtrationsschritten untersucht. Für den ersten Filtrationsschritt wurde eine Polykarbonatmembran mit einem Porendurchmesser von 2 µm verwendet, die prinzipiell geeignet wäre, Blutzellen von Plasma zu trennnen. Polykarbonat wurde verwendet, da dieses Filtermaterial eine sehr niedrige unspezifische Bindung von Liposomen an die Oberfläche des Filters aufweist. Im zweiten Filtrationsschritt wurde ein Zellulosemischesterfilter mit einem Porendurchmesser von 50 nm verwendet (beide Millipore GmbH, Eschborn, Deutschland), der die untersuchten Liposomen, die einen Durchmesser von etwa 110 nm aufwiesen, weitgehend zurückhalten sollte.

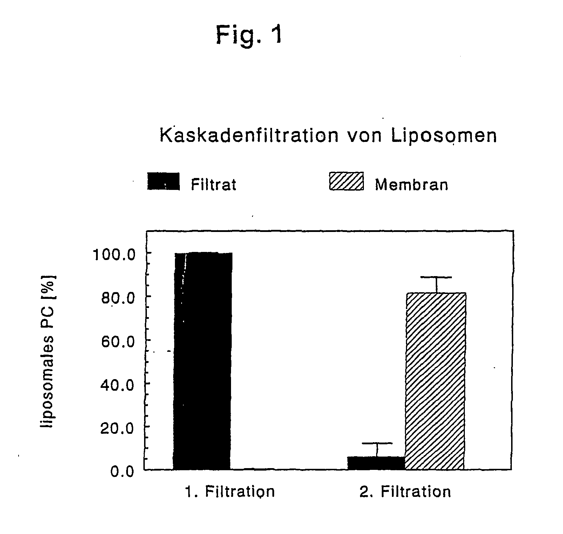

Die Abtrennung von Liposomen vom die Liposomen umgebenden Medium nach dem Prinzip der Kaskadenfiltration wurde anhand einer Liposomensuspension untersucht, die 0,5 mM liposomales PC enthielt. Für die Untersuchung wurden Liposomen verwendet, die 10 % DSPG enthielten und eine mittlere Größe von etwa 110 nm aufwiesen. Zur Filtration der Liposomen wurde eine modifizierte Form der Millipore-Filtriertechnik von G. Brierley und R.L. O'Brian: "Compartimentation of heart mitochondria", in J. Biol. Chem. 240 (1965), S. 4532-4539) angewandt. Es wurden Filter mit einem Filterdurchmesser von 25 mm verwendet. Die Filtration erfolgte in einem Vielfachfiltrationsgerät (Millipore GmbH, Eschborn, Deutschland). Das zur Filtration angelegte Vakuum wurde durch eine Membranpumpe erzeugt und betrug 900 mbar im ersten Filtrationsschritt und 100 mbar im zweiten Filtrationsschritt. Durch einen zwischen Membranpumpe und Filtrationseinheit liegenden Dreiwegehahn konnte das angelegte Vakuum reguliert werden. Der für die Membranfiltration verwendete Puffer entsprach dem bereits beschriebenen Liposomenpuffer.
Die mit Puffer angefeuchtete Polykarbonatmembran wurde in das Filtrationsgerät eingelegt und bei Umgebungsdruck mit 2 ml Liposomensuspension überschichtet und anschließend unter Unterdruck filtriert. Vom aufgefangenen Filtrat wurde 1 ml bei Umgebungsdruck auf eine mit Puffer angefeuchtete Zellulosemischestermembran gegeben. Anschließend wurde die Liposomensuspension erneut unter Vakuum filtriert. Zur Bestimmung der im Filtrat enthaltenen Mengen an liposomalem PC wurde dem Filtrat jeweils ein Aliquot entnommen. Die Membranen wurden nach erfolgter Filtration noch einmal mit 1 ml Puffer gespült, in ein Flüssigkeitsszintillationsgefäß überführt und durch Zugabe von 750 µl Biolute-S® (Zinsser Analytik GmbH, Frankfurt, Deutschland) aufgelöst. Die in den Filtraten und in den Membranen enthaltene Radioaktivität wurde anschließend wie oben beschrieben bestimmt.
Die erhaltenen Ergebnisse sind in Fig. 1 und in Tabelle 2 dargestellt. Es wurden 3 individuelle Messungen anhand einer Liposomenpräparation durchgeführt. In der Figur 1 dargestellt ist die Menge an liposomalem PC, die nach erfolgter Filtration im Filtrat oder auf der Filtermembran gemessen wurde.

**Tabelle 2**

| | Filtrat | Membran |
|---|---|---|
| 1. Filtration | 99,9 % (± 0,1 %) | 0,2 % (± 0,1 %) |
| 2. Filtration | 6,5 % (± 6,1 %) | 81,5 % (± 7,2 %) |

Während die Liposomen durch die Polykarbonatfiltermembran mit einem Porendurchmesser von 2 µm praktisch nicht zurückgehalten werden, können sie durch eine Zellulosemischestermembran mit einem Porendurchmesser von 50 nm weitgehend aufgefangen werden. Durch die Verwendung entsprechend ausgewählter Membranen und Adaption der Filtrationsbedingungen wie Flußgeschwindigkeiten und Filtrationsdrücken ist das Prinzip der Kaskadenfiltration somit grundsätzlich auch für die Abtrennung von Liposomen aus dem Blut anwendbar.

### 4. Bezugsbeispiel 2 - Fällung von DSPE-haltigen Liposomen

Zum prinzipiellen Nachweis einer Interaktion von Liposomen, die geladene Gruppen auf ihrer Oberfläche tragen, mit entsprechend gegensinnig geladenen Polyionen, und zum prinzipiellen Nachweis der aus dieser Wechselwirkung resultierenden Fällbarkeit von entsprechenden Liposomen, wurden Heparin als Polyanion und Liposomen, die 5 Mol% DSPE enthielten, verwendet. Eine Liposomensuspension, die 0,3 mM liposomales PC in Liposomenpuffer enthielt, wurde mit dem gleichen Volumen eines 0,2 mM Acetatpuffers pH 4,85, der 100 i.E. Heparin/ml und 5 mM CaCl₂ enthielt, bei RT vermischt. Das Gemisch wurde 5 min lang ruhen gelassen und anschließend 10 min lang bei 12000 × g zentrifugiert. Der Überstand wurde vom Niederschlag getrennt, und der Niederschlag wurde in Ca²⁺-freiem Liposomenpuffer, der 5 mM EGTA enthielt, dispergiert.

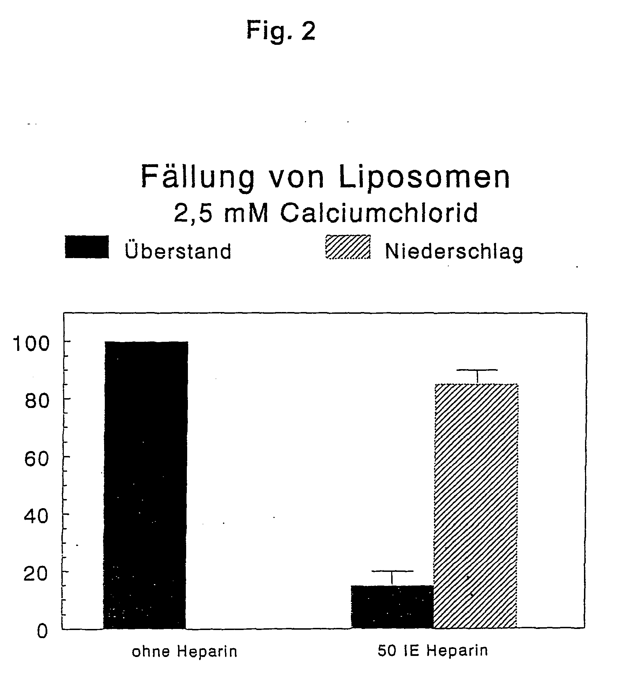

Zur Bestimmung der Menge an Liposomen im Überstand und im Niederschlag wurde die Radioaktivität im Überstand und im Niederschlag quantifiziert. Zur Überprüfung der Integrität der präzipitierten Liposomen wurde die Größe der Liposomen vor der Fällung und nach der Fällung untersucht.
Die Ergebnisse der Fällung sind in Fig. 2 dargestellt und werden in Tabelle 3 und in Tabelle 4 quantifiziert, wobei anhand einer Liposomensuspension 3 individuelle Fällungen durchgeführt wurden. In der Fig. 2 sind die Mittelwerte aufgetragen.

**Tabelle 3: Verteilung von liposomalem PC nach der Präzipitation von DSPE-Liposomen**

| | Überstand | Niederschlag |
|---|---|---|
| Liposomales PC | 14,8 % (± 5 %) | 85,2 % (± 5%) |

**Tabelle 4: Durchmesser der Liposomen vor und nach Ihrer Präzipitation**

| vor Fällung | nach Fällung |
|---|---|
| 131,9 nm (± 1,0 nm) | 136,6 nm (± 0,1 nm) |

Mit Hilfe der ermittelten Daten konnte gezeigt werden, dass Liposomen die positiv geladene Gruppen auf ihrer Oberfläche tragen, prinzipiell durch ein Polyanion in Gegenwart von Ca²⁺ präzipitiert werden können. Eine Fällung erfolgte nur in Gegenwart von Heparin als polyanionischem Fällungsmittel. Die Fällung der Liposomen erfolgte unter Bedingungen, die auch bei der HELP-Apherese angewandt werden. Unter diesen Bedingungen ist die Fällung sehr effizient. Die Fällung der Liposomen ist prinzipiell reversibel, da die Liposomen nach erfolgter Zentrifugation resuspendiert werden können. Da die Liposomen nach der Fällung praktisch die gleiche Größe wie vor der Fällung aufwiesen, ist davon auszugehen, dass die Liposomen während der Fällung stabil bleiben und eventuell in ihrem Innern verkapselt vorliegende toxische Stoffe nicht freigesetzt werden.

### 5. Bezugsbeispiel 3 - Fällung und Filtration von Liposomen die eine positive Nettoladung tragen

Im gemäß Bezugsbeispiel 2 beschriebenen Versuch konnte gezeigt werden, dass Liposomen, die auf ihrer Oberfläche positiv geladene Gruppen tragen, prinzipiell mit Polyanionen präzipitiert werden können. Beim HELP-Verfahren werden die präzipitierten Komplexe aus Heparin und Lipoproteinen jedoch nicht durch Zentrifugation sondern durch Filtration des entsprechenden Serums durch eine Polykarbonatmembran mit einem Porendurchmesser von 450 nm entfernt. Um herauszufinden, ob die Komplexe aus Heparin und Liposomen analog dem HELP-Verfahren filtrierbar sind, wurde nach erfolgter Präzipitation der Liposomen ein Filtrationsschritt zur Separation der Liposomen vom umgebenden Medium untersucht. Für die Versuche wurden Liposomen verwendet, die 5 % DPDAP oder 5 % DPTAP enthielten.

Die Liposomen wurden wie im Bezugsbeispiel 2 beschrieben präzipitiert. Anstelle der Zentrifugation wurde nun jedoch 1 ml der das Präzipitat enthaltenen Lösung nach dem im Bezugsbeispiel 1 beschriebenen Verfahren filtriert. Für die Filtration wurden Polykarbonatfilter mit einem Porendurchmesser von 400 nm (Millipore) verwendet. Die Filtration erfolgte bei einem Vakuum von etwa 900 mbar. Von den aufgefangenen Filtraten wurde zur Bestimmung der enthaltenen Menge an liposomalem PC wiederum jeweils ein Aliquot entnommen, und die Filtermembranen wurden wie bereits beschrieben behandelt, um die auf ihnen zurückgebliebene Menge an liposomalem PC zu bestimmen.

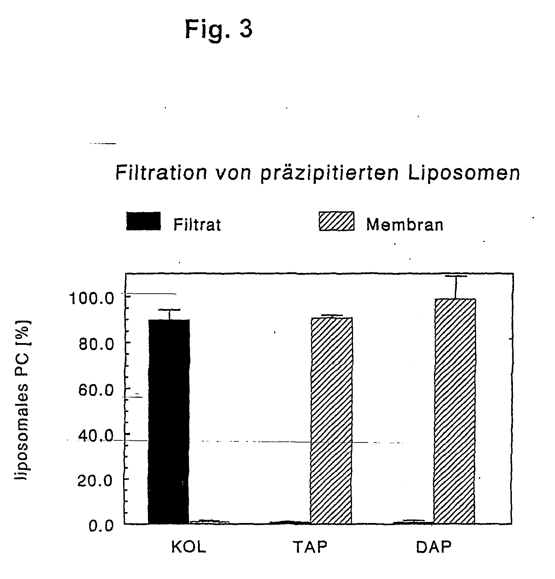

Die erhaltenen Ergebnisse sind in Fig. 3 und in Tabelle 4 dargestellt. Die Abkürzungen bedeuten:
KOL: Liposomen, die keine Ladung trugen
TAP: Liposomen, die 5 Mol% permanent positiv geladene Lipide enthielten,
DAP: Liposomen, die 5 Mol% eines protonierbaren Lipides enthielten.
Anhand einer Liposomenpräparation wurden zwei individuelle Messungen durchgeführt.

**Tabelle 4: Filtration von präzipierten Liposomen**

| | Filtrat | Membran |
|---|---|---|
| KOL | 89,9 % (± 4,3 %) | 1, 0 % (± 0,5 %) |
| TAP | 1,1 % (± 0,1 %) | 90,6 % (± 1,3 %) |
| DAP | 1,1 % (± 0,6 %) | 98,9 % (± 9,9 %) |

Liposomen, die eine positive Nettoladung tragen, können durch Heparin präzipitiert werden und gemäß den Bedingungen, die beim HELP-Verfahren angewandt werden, filtriert werden. Dementsprechend ist das für die HELP-Apherese angewandte Verfahren nach entsprechender Adaption grundsätzlich für die Elemination von Liposomen aus dem Blut geeignet.

Das HELP-Verfahren arbeitet mit einer pH-Absenkung im Extrakorporalkreislauf. Dies erlaubt die Verwendung von Liposomen, die Lipide tragen, die bei physiologischem pH ungeladen sind, bei niedrigerem pH jedoch protoniert und anschließend präzipitiert werden können. Ein solches Lipid könnte z.B. das in diesem Beispiel verwendete DPDAP sein. Bei der Verwendung von Liposomen, die bei physiologischem pH eine permanente positive Nettoladung tragen, kann eventuell auf eine pH-Absenkung im Extrakorporalkreislauf verzichtet werden.

### 6. Bezugsbeispiel 4 - Adsorptionschromatographie von Liposomen

Das DALI-Verfahren arbeitet mit einer Kombination aus Gelpermeations- und Adsorptionschromatographie. Um Nachzuweisen, dass Liposomen grundsätzlich durch Adsorptionschromatographie aus einer Liposomensuspension entfernt werden können, wurden Liposomen die entweder eine negative Nettoladung oder eine positive Nettoladung trugen, über eine Chromatographiesäule, die an Sephadex gebundenenes Heparin enthielt, chromatographiert. Verwendet wurden DSPGhaltige Liposomen oder DPDAP-haltige Liposomen. Die Chromatographie erfolgte mit Hilfe eines FPLC^{®}-Systems (Pharmacia, Freiburg, Deutschland). Die verwendete Chromatographiesäule wies einen Durchmesser von 16 mm und eine Länge von 35 mm auf, die Flußgeschwindigkeit betrug 0,5 ml/min. Aufgetragen_wurden 50 µl einer 0,5 mM liposomales PC enthaltenen Liposomensuspension. Es wurden Fraktionen zu jeweils 1 ml aufgefangen und die in ihnen enthaltene Radioaktivität wurde wie oben beschrieben bestimmt. Für die Chromatographie wurde ein 0,2 mM Acetatpuffer pH 4,5 oder ein Puffer aus 10 mM Hepes und 130 mM NaCl pH 9,0 verwendet.

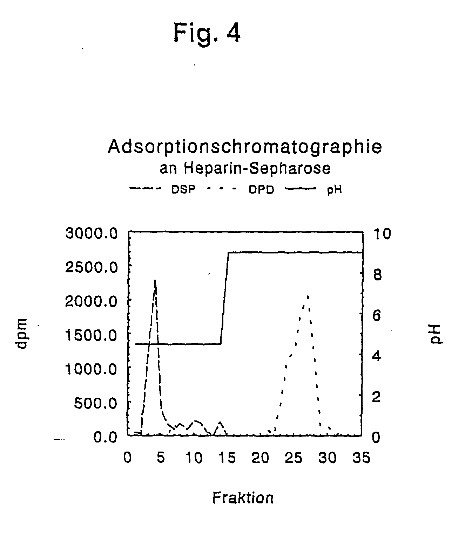

Der Verlauf der Chromatographie ist in Fig. 4 dargestellt. Während die DSPG-haltigen Liposomen, die eine negative Nettoladung tragen, praktisch nicht mit dem heparinhaltigen Säulenmaterial in Wechselwirkung treten und innerhalb eines Säulenvolumens eluieren, werden die DPDAP-haltigen Liposomen, die bei pH 4,5 eine positive Nettoladung tragen, vollständig vom Säulenmaterial adsorbiert. Durch eine Änderung des pH auf 9,0 werden die DPDAP-haltigen Liposomen protoniert und konnten eluiert werden.

Liposomen können somit durch eine geeignete Wechselwirkung, z.B. elektrostatischer Natur, mit Hilfe einer Adsorptionschromatographie vom sie umgebenden Medium getrennt werden. Der Extrakorporalkreislauf erlaubt hierbei eine Adaption der Bedingungen für die optimale Bindung der Liposomen an das Adsorptionsmaterial.

## Patentansprüche

1. Verwendung von potentiell toxischen und/oder schädlichen therapeutischen Wirkstoff in Form eines partikulären Trägersystems enthaltenden Partikeln, die zum Binden, zur Aufnahme und/öder zum Tragen des toxischen und/oder schädlichen Wirkstoffs geeignet sind und ausgewählt sind aus Liposomen, Mikrosphären, Nanopartikeln, Niosomen oder Polymerpartikeln, zur Herstellung eines Medikaments zur Verminderung der Toxizität des Wirkstoffs, wobei der therapeutische Wirkstoff in Form des partikulären Trägersystems appliziert und nach Erreichen des Wirkoptimums über den partikulären Träger in einem extrakorporalen Schritt oder in einer körperfremden Einrichtung aus einer Körperflüssigkeit zumindest zum Teil wieder entfernt wird.

2. Verwendung gemäß Anspruch 1, wobei die therapeutischen Wirkstoffe aus der Gruppe ausgewählt sind, die aus Cytostatika, Antibiotika, Antivirusmitteln, Antimykotika, in der Gentherapie eingesetzte Substanzen, Antikörpern, Interferonen, Cytokinen und Radionuklide besteht.

3. Verwendung gemäß Anspruch 1 oder 2, wobei die Wirkstoff enthaltenden oder tragenden Partikel nach der Entfernung aus der Körperflüssigkeit wiedergewonnen werden.

4. Verwendung gemäß einem der vorangehenden Ansprüche, **dadurch gekennzeichnet, daß** eine vollständige oder teilweise Entfernung zur Dosiseinstellung oder zur Einstellung eines zeitlichen Therapieschemas eines therapeutischen Wirkstoffs durchgeführt wird.

5. Verwendung nach Anspruch 4, **dadurch gekennzeichnet, dass** zur jeweiligen Einstellung der Gehalt des entfernten Wirkstoffs und/oder des Partikels bestimmt wird.

## Claims

1. Use of potentially toxic and/or harmful therapeutic agent in the form of a particulate carrier system comprising particles, which are capable of binding, taking up and/or carrying the toxic and/or harmful therapeutic agent and which are selected from liposomes, microspheres, nanoparticles, niosomes or polymer particles, for the manufacture of a medicament for reducing the toxicity of the therapeutic agent, wherein the therapeutic agent is applied in form of the particulate carrier system and, after reaching the optimum of activity, is at least partially removed again from a body fluid via the particulate carrier in an extracorporeal step or in an exogenous device.

2. Use according to claim 1, wherein the therapeutic agent is selected from the group consisting of cytostatic agents, antibiotics, antiviral agents, antimycotic agents, substances used in gene therapy, antibodies, interferons, cytokines and radionuclides.

3. Use according to claim 1 or 2, wherein the particles containing or carrying the therapeutic agent are recovered after removal from the body fluid.

4. Use according to any one of the proceeding claims, **characterized in that** a complete or partial removal is carried out for dosing or for adjusting a therapeutic timing of a therapeutic agent.

5. Use according to claim 4, **characterized in that** the content of the removed therapeutic agent and/or particle is determined for the respective adjustment.

## Revendications

1. Utilisation de particules comportant un agent thérapeutique potentiellement toxique et / ou nocif sous la forme d'un système de vecteurs de particules , lesquels particules sont aptes à lier, recevoir et / ou véhiculer l'agent toxique et / ou nocif et qui ont été choisis parmi des liposomes, microsphères, nanoparticules, niosomes, ou particules polymériques pour fabriquer un médicament pour réduire la toxicité de l'agent, où l'agent thérapeutique est appliqué sous la forme d'un système de vecteurs et est enlevé au moins partiellement d'un liquide corporel après avoir atteint l'optimum d'effet via le vecteur de particules dans une étape extracorporel ou dans une installation exogène.

2. Utilisation selon la revendication 1, où les agents thérapeutiques ont été choisis parmi le groupe qui est composé de cytostatiques, d'antibiotiques, de remèdes antiviraux, des antimicotiques , de substances employées dans la thérapie génétique, des cytokines et ou des radionucléides.

3. Utilisation selon les revendications 1 ou 2, où les particules comportant ou véhiculant l'agent sont récupérées après l'enlèvement du liquide corporel.

4. Utilisation selon une des revendications précédentes, **caractérisée en ce qu'**un enlèvement complet ou partiel est effectué pour régler la dose ou pour régler un schéma thérapeutique temporel d'un agent thérapeutique.

5. Utilisation selon la revendication 4, **caractérisé en ce que** l'on détermine pour le réglage respectif le contenu de l'agent enlevé et / ou de la particule.
